# FASCICULE DE BREVET EUROPEEN

(11) **EP 0 716 093 B1**
(45) Date de publication et mention de la délivrance du brevet: **21.10.1998**
(21) Numéro de dépôt: 95402744.7
(22) Date de dépôt: 06.12.1995
(51) Int. Cl.: C07H 17/08, A61K 31/70

(54) **Nouveaux dérivés de l'érythromycine, leur procédé de préparation et leur application comme médicaments**
Erythromycin-Derivate, Verfahren zur Herstellung und Verwendung als Arzneimittel
Erythromycin derivatives, their process of preparation and application as medicaments

(30) Priorité: 09.12.1994 FR 9414807
(43) Date de publication de la demande: 12.06.1996
(73) Titulaire: HOECHST MARION ROUSSEL, 92800 Puteaux (FR)
(72) Inventeur: Agouridas, Constantin, F-94130 Nogent sur Marne (FR); Chantot, Jean-François, F-94130 Nogent sur Marne (FR)
(74) Mandataire: Vieillefosse, Jean-Claude

(56) Documents cités:
- EP-A- 0 487 411
- EP-A- 0 596 802
- EP-A- 0 606 024
- EP-A- 0 606 062
- FR-A- 2 692 579

## Description

La présente invention concerne de nouveaux dérivés de l'érythromycine, leur procédé de préparation et leur application comme médicaments.

L'invention a pour objet les composés de formule (I) : 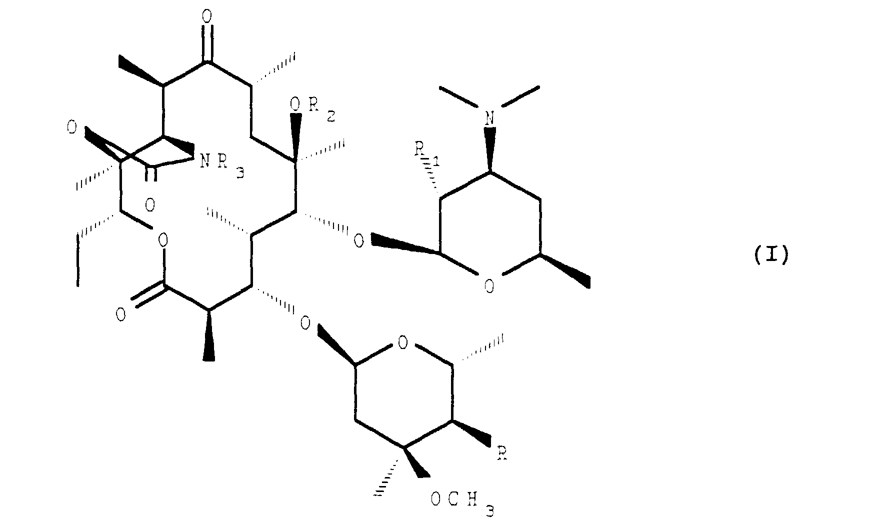 dans lesquels
R et R₁ représentent un radical hydroxyle ou O-acyle renfermant de 2 à 20 atomes de carbone,
R₂ est un atome d'hydrogène ou un radical méthyle,
R₃ représente un radical -N-(CH₂)_{q}R₄ dans lequel q représente un nombre entier de O à 6, R₄ étant un radical hétérocyclique mono ou polycyclique éventuellement substitué, ainsi que leurs sels d'addition avec les acides.

Comme exemple de sels d'addition des présents dérivés avec les acides minéraux ou organiques, on peut citer les sels formés avec les acides acétique, propionique, trifluoroacétique, maléique, tartrique, méthanesulfonique, benzènesulfonique, p-toluènesulfonique, chlorhydrique, bromhydrique, iodhydrique, sulfurique, phosphorique et spécialement les acides stéarique, éthylsuccinique et laurylsulfonique.

Le radical acyle est de préférence un radical acétyle, propionyle, butyryle, isobutyryle, n-valéryle, isovaléryle, tert-valéryle, pivalyle ou phénylméthoxycarbonyle.

Le radical hétérocyclique comporte un ou plusieurs hétéroatomes choisis de préférence parmi l'oxygène, le soufre et l'azote.

Le radical hétérocyclique peut être un radical à 5 chaînons, de préférence le radical thiényle, furyle, pyrolyle, thiazolyle, oxazolyle, imidazolyle, thiadiazolyle, pyrazolyle, isoxazolyle ou triazolyle,
le radical hétérocyclique peut être un radical à 6 chaînons, de préférence un radical pyridyle, pyrimidinyle, pyridazinyle ou pyrazinyle,
le radical hétérocyclique peut être également un radical condensé comme par exemple le radical benzimidazolyle, indolyle, benzofurannyle, benzothiazolyle ou quinoléinyle.

Lorsque le radical hétérocyclique est substitué, il l'est de préférence par un ou plusieurs substituants choisis parmi les atomes d'halogène, les radicaux hydroxy, alkyle, alkyloxy, aryle, aryloxy renfermant jusqu'à 18 atomes de carbone.

L'invention a particulièrement pour objet les composés de formule (I) dans lesquels R₃ est un radical N(CH₂)_{q}R₄, q et R₄ conservant leur signification précédente et notamment dans lesquels q représente la valeur 3.

L'invention a plus particulièrement pour objet les composés de formule (I) dans lesquels le radical hétérocycle R₄ renferme au moins un atome d'azote, notamment ceux dans lesquels le radical hétérocyclique est un radical imidazolyle, pyridinyle, thiazolyle, quinoléinyle ou azabenzimidazolyle, éventuellement substitué et plus spécialement un radical 4-phényl 1H-imidazolyle ou 4-quinoléinyle.

L'invention a tout particulièrement pour objet les composés de formule (I) dont la préparation est donnée ci-après dans la partie expérimentale et tout spécialement les produits des exemples 1, 2, 3 et 4.

L'invention a également pour objet un procédé de préparation des composés de formule (I), caractérisé en ce que l'on soumet un composé de formule (II) : 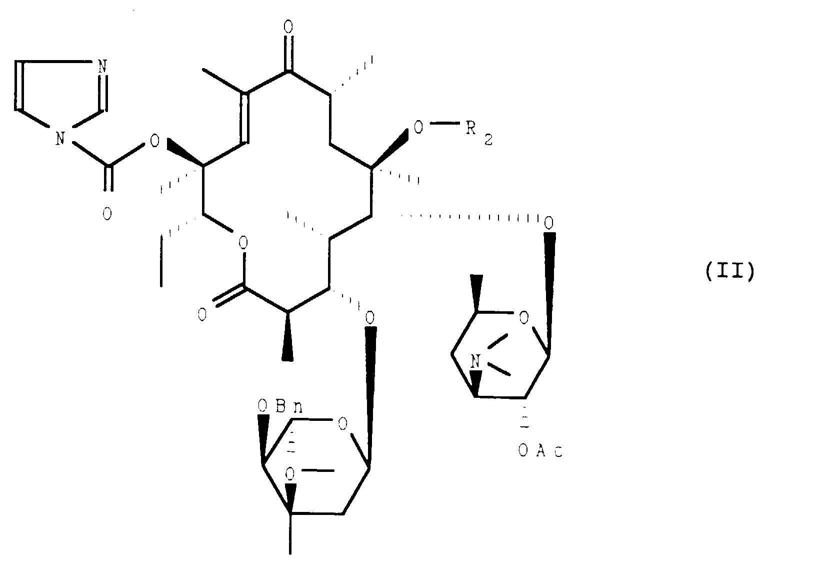 dans laquelle R₂ conserve sa signification précédente, Bn représente un radical benzyloxycarbonyle et Ac un radical acyle tel que défini ci-dessus, à l'action d'un composé de formule (III) :

R₃NH₂ (III)

dans laquelle R₃ conserve sa signification précédente pour obtenir les composés de formule (IV) : 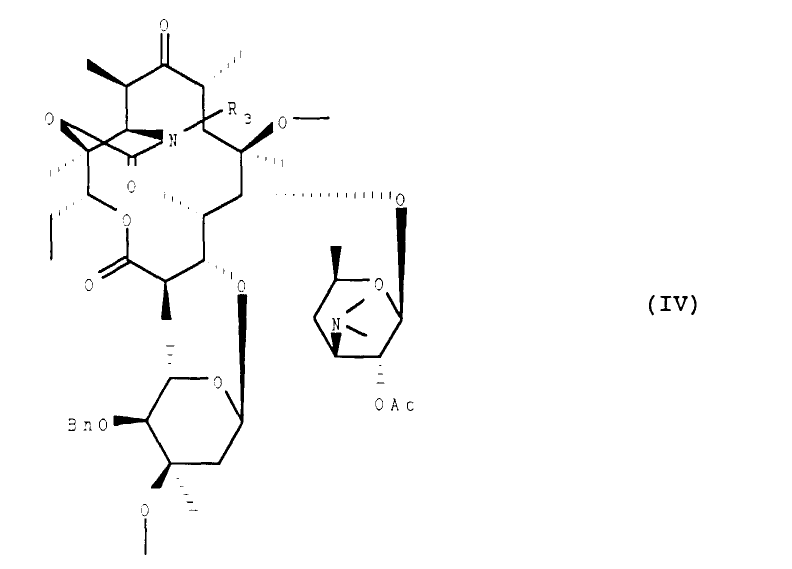 que l'on soumet si désiré à l'action d'un agent de clivage de la fonction ester en 2' pour obtenir le composé de formule (I) dans lequel R₁ est un radical OH, puis soumet si désiré le composé ainsi obtenu à l'action d'un agent de réduction pour effectuer le clivage du groupement benzyloxy carbonyle en 4" et obtenir le produit de formule (I) dans lequel R représente un radical OH puis, si désiré, soumet le composé de formule (I) à l'action d'un acide pour en former le sel correspondant.

Les composés de formule (II) utilisés comme produits de départ sont des produits connus décrits dans le brevet 0 248 279.

Les amines de formule (III) sont connus d'une façon générale et peuvent être préparés selon les procédés décrits dans J. Med. Chem. (1982) vol. 25, p. 947 et suivantes ou encore Tetrahedron Letters vol. 32 n° 14, p. 1699, 1702 (1991).

Le clivage de l'acétate en 2' est réalisé à l'aide du méthanol ou de l'acide chlorhydrique aqueux.

Le clivage du groupe benzyloxycarbonyle en 4" est réalisé par réduction, par exemple au moyen de l'hydrogène en présence d'un catalyseur au palladium.

La salification est réalisée au moyen d'acide selon les procédés classiques.

Les composés de formule (I) dans lesquels R₃ est un radical -N(CH₂)_{q}R₄ peuvent être préparés par action de l'hydrate d'hydrazine sur le produit de formule (II) pour obtenir le composé de formule (P), c'est-à-dire un produit de formule (I), dans lequel R₃ représente NH₂, que l'on soumet à l'action d'un aldéhyde R₄(CH₂)_{q-1}CHO, pour obtenir le composé de formule (I) correspondant. Le composé de formule (P) est un produit de l'invention à titre de produit chimique nouveau.

Les produits de formule générale (I) possèdent une très bonne activité antibiotique sur les bactéries gram ^{⊕} telles que les staphylocoques, les streptocoques, les pneumocoques.

Les composés de l'invention peuvent donc être utilisés comme médicaments dans le traitement des infections à germes sensibles et notamment, dans celui des staphylococcies, telles que les septicémies à staphylocoques, staphylococcies malignes de la face ou cutanées, pyodermites, plaies septiques ou suppurantes, furoncles, anthrax, phlegmons, érysipèles et acné, staphylococcies telles que les angines aiguës primitives ou post-grippales, bronchopneumonies, suppuration pulmonaires, les streptococcies telles que les angines aiguës, les otites, les sinusites, la scarlatine, les pneumococcies telles que les pneumonies, les bronchites ; la brucellose, la diphtérie, la gonococcie.

Les produits de la présente invention sont également actifs contre les infections dues à des germes comme Haemophilus influenzae, Moraxella catarrhalis, Rickettsies, Mycoplasma pneumoniae, Chlamydia, Legionella, Ureaplasma, Toxoplasma ou à des germes du genre Mycobactérium.

La présente invention a donc également pour objet, à titre de médicaments et, notamment de médicaments antibiotiques, les produits de formule (I) tels que définis ci-dessus, ainsi que leurs sels d'addition avec les acides minéraux ou organiques pharmaceutiquement acceptables.

L'invention a plus particulièrement pour objet, à titre de médicaments et, notamment de médicaments antibiotiques, les produits préférés de formule (I) définis précédemment à savoir les produits des exemples 1, 2, 3 et 4, ainsi que leurs sels pharmaceutiquement acceptables.

L'invention a également pour objet les compositions pharmaceutiques renfermant comme principe actif au moins un des médicaments défini ci-dessus.

Ces compositions peuvent être administrées par voie buccale, rectale, parentérale ou par voie locale en application topique sur la peau et les muqueuses, mais la voie d'administration préférée est la voie buccale.

Elles peuvent être solides ou liquides et se présenter sous les formes pharmaceutiques couramment utilisées en médecine humaine, comme par exemple, les comprimés, simples ou dragéifiés, les gélules, les granulés, les suppositoires, les préparations injectables, les pommades, les crèmes, les gels ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent également se présenter sous forme d'une poudre destinée à être dissoute extemporanément dans un véhicule approprié, par exemple de l'eau stérile apyrogène.

La dose administrée est variable selon l'affection traitée, le sujet en cause, la voie d'administration et le produit considéré. Elle peut être, par exemple, comprise entre 50 mg et 300 mg par jour par voie orale, chez l'adulte pour le produit de l'exemple 4 ou 2.

Les exemples suivants illustrent l'invention sans toutefois la limiter.

### EXEMPLE 1 : 11,12-didéoxy 6-O-méthyl 12,11-(oxycarbonyl-(2-(3-(4-quinoléinyl) propyl) hydrazono) érythromycine

### STADE A : Préparation de 11,12-didéoxy 6-O-méthyl 12,11-(oxycarbonyl (2-hydrazono)) érythromycine (produit P)

### a) Condensation

On plonge dans un bain à 80°C durant 10 min, un mélange de 3 g de 2'-acétate 12-(1H-imidazole-1-carboxylate 4"-(phénylméthylcarbonate) de 10,11-didéhydro 11-déoxy 6-O-méthyl érythromycine, 3 ml d'hydrate d'hydrazine, 30 ml d'acétonitrile et 492 mg de carbonate de césium. On concentre à sec, reprend au chlorure de méthylène, lave à l'eau, sèche, filtre et amène à sec.

### b) Désacétylation

On dissout le produit obtenu (3 g) dans 30 ml de méthanol et agite à la température ambiante pendant 15 heures et concentre à sec. On obtient 2,7 g de produit désacétylé.

### c) Hydrogénolyse

On dissout le produit obtenu au stade b) dans 100 ml de méthanol. On hydrogénolyse en présence de 600 mg de palladium à 10 % sur charbon actif. On filtre, rince au méthanol et au chlorure de méthylène puis concentre à sec le filtrat. On obtient 2,52 g d'un produit que l'on purifie en éluant avec le mélange éther isopropylique-méthanol-triéthylamine (80-10-10). On obtient 941,8 mg d'un produit que l'on chromatographie à nouveau en éluant avec le mélange éther isopropylique-méthanol-triéthylamine (80-10-10). On obtient ainsi 761 mg de 6-O-méthyl-12,11-(oxycarbonyl) (2-hydrazono)) érythromycine.

| Microanalyse | Calculé | Trouvé |
|---|---|---|
| C % | 59,45 | 58,8 |
| H % | 8,83 | 8,5 |
| N % | 5,33 | 5,2 |

### Spectre de masse

788⁺ = MH⁺
810⁺ = MNa⁺

### RMN : CDCl₃ 300 MHz ppm

- CH₃-CH₂: 0,84 (t)
- les CH₃-CH: 1,08 (d)-1,11 (d)-1,14 (d) 1,16 (d)-1,21 (d)-1,23 (d)

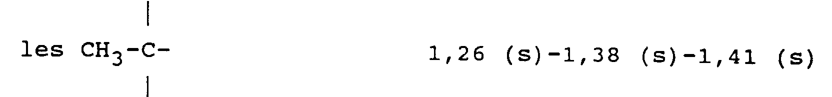

- N(Me)₂: 2,28 (s)
- H'₃: ~ 2,40 (m)
- H₂: 2,88 (m)
- H₈: 2,66 (m)
- H"₄: ~ 3,00
- les 6-OMe: 3,02 (s)
- H₁₀: ~ 3,08
- H'₂: 3,18 (dd)
- 3"-OMe: 3,33 (s)
- H'₅: 3,48 (m)
- H₁₁: 3,60 (s)
- H₃ et H₅: 3,65 (d)
- H"₅: 4,00 (m)
- H'₁: 4,43 (d)
- H₁₂: 4,50 (s)
- H"₁: 4,91 (d)
- H₁₃: 5,02 (dd)

### STADE B : 11,12-didéoxy 6-O-méthyl 12,11-(oxycarbonyl-(2-(3-(4-quinoléinyl) propyl) hydrazono) érythromycine

On agite 15 heures à la température ambiante un mélange de 230 mg du produit obtenu au stade A ci-dessus, 5 ml de méthanol, 0,3 g de quinoléine 4-propanal, 0,055 ml d'acide acétique. On ajoute 0,065 g de cyanoborohydrure de sodium. On agite à la température ambiante pendant 24 heures. On évapore le méthanol, extrait à l'acétate d'éthyle, lave à l'aide d'une solution de soude puis à l'eau, sèche, filtre et évapore à sec. On obtient 220 mg d'un produit que l'on chromatographie sur silice en éluant avec le mélange acétate d'éthyle-triéthylamine (95-5). On obtient 80 mg du produit recherché.

| Microanalyse | Calculé | Trouvé |
|---|---|---|
| C % | 63,99 | 64,1 |
| H % | 8,42 | 8,3 |
| N % | 5,85 | 5,7 |

### Spectre de masse

158⁺ = OH en 2'
957⁺ = (M+H)⁺
979⁺ = (M⁺Na)⁺

### RMN : CDCl₃ 400 MHz ppm

- CH₃-15: 0,78 (t)
- CH₃-10: 1,07 (d)
- CH₃-4: 1,11 (d)
- CH₃-8: 1,16 (d)
- CH₃-5' et CH₃-2: 1,22 (d)
- CH₃-5": 1,32 (d)

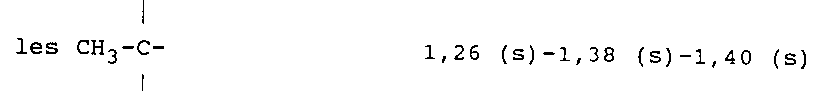

- CH₂-14: 1,52 (m)- ~1,90 (m)
- CH₂-2": 1,62 (dd, J = 15 et 5) 2,38 (d, J = 15)
- H₄: ~ 1,87 (m)
- OH-4": 2,20 (d large, mobile)
- N(CH₃)₂: 2,29 (s)
- H'₃: 2,42 (m)
- H₈: 2,65 (m)
- H₂: 2,94 (m)
- OCH₃-6: 2,99 (s)
- H"₄: 3,03 (m)
- H₁₀ et H'₂: ~ 3,17 (m)
- OCH₃-3": 3,33 (s)
- CH₂-O et CH₂-N: 2,90 à 3,35 (m)
- H'₅: 3,49 (m)
- H₅: 3,67 (d, J = 7)
- H₃: 3,70 (d, J = 10)
- H₁₁: 3,76 (s)
- H"₅: 4,01 (m)
- H'₁: 4,43 (d, J = 7)
- H"₁: 4,94 (dl, J = 5)
- H₁₃: 4,98 (dd, J = 11 et 2)
- NH-CH₂: 5,63 (m, mobile)
- H₃: 7,30 (d, J = 4)
- H₂: 8,78 (d, J = 4)
- H₆, H₇: 7,52 (dt)-7,66 (dt)
- H₅, H₈: 8,07 (dl)-8,12 (dl)

### EXEMPLE 2: 11,12-didéoxy 6-O-méthyl 12,11-(oxycarbonyl (2-(3-(4-phényl-1H-imidazol-1-yl) propyl) hydrazono)) érythromycine.

rf = 0,1 (éther isopropylique-méthanol-triéthylamine 80-10-10).

### EXEMPLE 3: 6-O-méthyl 12,11-(oxycarbonyl (2-(3-(3H-imidazo (4,5-b)-pyridin-3-yl) propyl) hydrazono)) érythromycine.

rf = 0,2 (éther isopropylique-méthanol-triéthylamine 80-10-10).

### EXEMPLE 4: 6-O-méthyl 12,11-(oxycarbonyl (2-(3-(2-phényl 4-thiazolyl) propyl) hydrazono)) érythromycine.

rf = 0,14 (éther isopropylique-méthanol-triéthylamine 80-10-10).

### EXEMPLE DE COMPOSITION PHARMACEUTIQUE

On a préparé le composé renfermant :

| | |
|---|---|
| **Produit de l'exemple 4** | 150 mg |
| Excipient q.s.p. | 1 g |
| Détail de l'excipient : amidon, talc, stéarate de magnésium | |

### ETUDE PHARMACOLOGIQUE DES PRODUITS DE L'INVENTION

### Méthode des dilutions en milieu liquide

On prépare une série de tubes dans lesquels on répartit une même quantité de milieu nutritif stérile. On distribue dans chaque tube des quantités croissantes du produit à étudier, puis chaque tube est ensemencé avec une souche bactérienne.

Après incubation de vingt-quatre heures à l'étuve à 37°C, l'inhibition de la croissance est appréciée par transillumination de ce qui permet de déterminer les concentrations minimales inhibitrices (C.M.I.) exprimées en microgrammes/cm³.

Les résultats suivants ont été obtenus :

| Souches bactériennes à GRAM⁺ | |
|---|---|
| Produits | Ex. 4 |
| Staphylococcus aureus 011UC4 | 0,3 |
| Streptococcus pyogenes | 0,08 |
| groupe A 02A1UC1 | |
| Streptococcus agalactiae | ≤0,02 |
| groupe B 02B1HT1 | |
| Streptococcus faecalis | 0,08 |
| groupe D 02D2UC1 | |
| Streptococcus faecium | 0,08 |
| groupe D 02D3HT1 | |
| Streptococcus sp | 0,08 |
| groupe G 02G0GR5 | |
| Streptococcus agalactiae | 5 |
| groupe B 02B1SJ1 | |

## Revendications

1. Les composés de formule (I) : 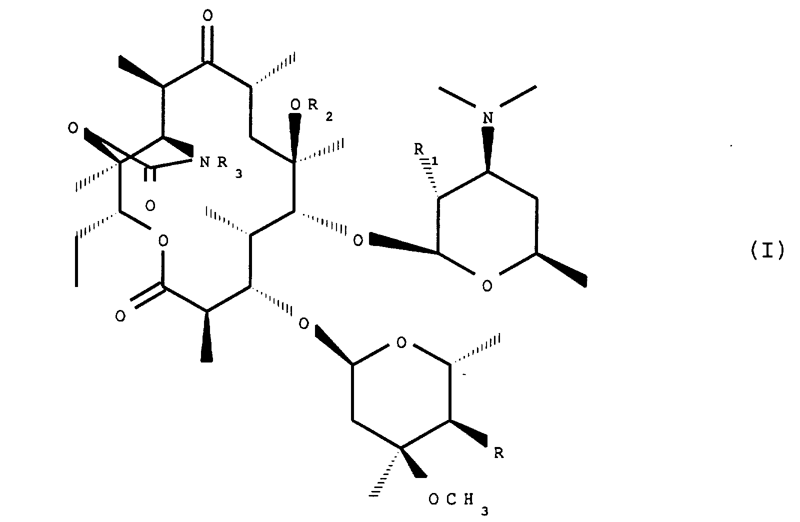 dans lesquels -
R et R₁ représentent un radical hydroxyle ou O acyle renfermant de 2 à 20 atomes de carbone,
R₂ est un atome d'hydrogène ou un radical méthyle,
R₃ représente un radical -N-(CH₂)_{q}R₄ dans lequel q représente un nombre entier de 0 à 6, R₄ étant un radical hétérocyclique mono ou polycyclique éventuellement substitué, ainsi que leurs sels d'addition avec les acides.

2. Les composés de formule (I) tels que définis à la revendication 1, dans lesquels R et R₁ représentent un radical hydroxyle.

3. Les composés de formule (I) tels que définis à la revendication 1 ou 2, dans lesquels R₂ est un radical méthyle.

4. Les composés de formule (I) tels que définis à la revendication 3 dans lesquels q représente la valeur 3.

5. Les composés de formule (I) tels que définis à l'une quelconque des revendications 1 à 4, dans lesquels le radical hétérocyclique R₄ renferme au moins un atome d'azote.

6. Les composés de formule (I) tels que définis à la revendication 5, dans lesquels le radical hétérocyclique est choisi dans le groupe constitué par les radicaux imidazolyle, pyridinyle, thiazolyle, quinoléinyle et azabenzimidazolyle, éventuellement substitués.

7. Les composés de formule (I) tels que définis à la revendication 9, dans lesquels R₄ représente un radical 4-phényl-1H-imidazolyle ou 4-quinoléinyle.

8. Le composé de formule (I) dont le nom suit :
- 11,12-didéoxy 6-O-méthyl 12,11-(oxycarbonyl-(2-(3-(4-quinoléinyl) propyl) hydrazono)) érythromycine.

9. Procédé de préparation des composés de formule (I) tels que définis à l'une quelconque des revendications 1 à 8, caractérisé en ce que l'on soumet un composé de formule (II): 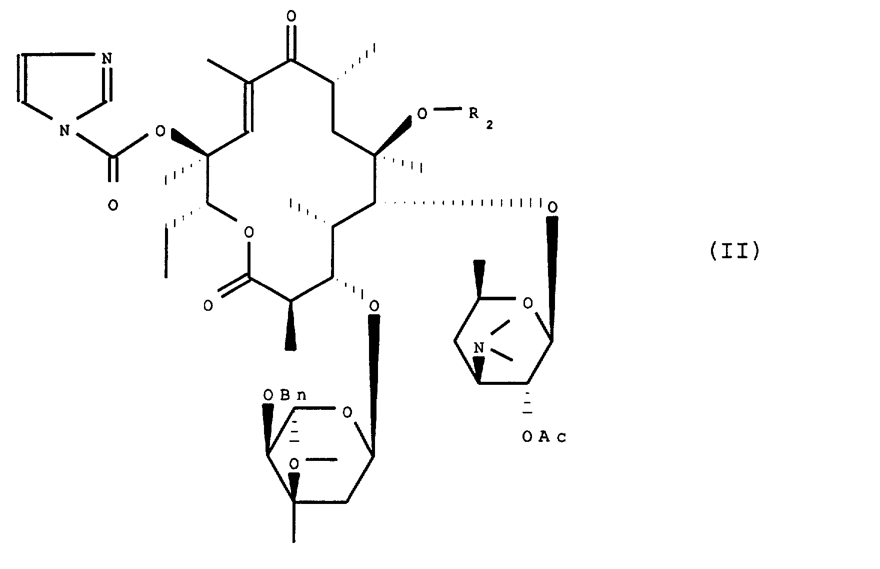 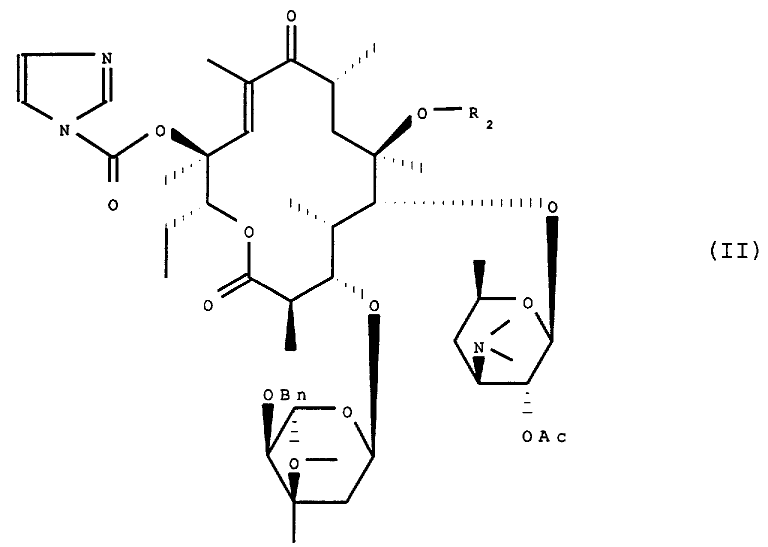 dans laquelle R₂ conserve sa signification précédente, Bn représente un radical benzyloxycarbonyle et Ac un radical acyle, tel que défini à la revendication 1 à l'action d'un composé de formule (III) :
R₃NH₂ (III)
dans laquelle R₃ conserve sa signification précédente pour obtenir le composé de formule (IV) : 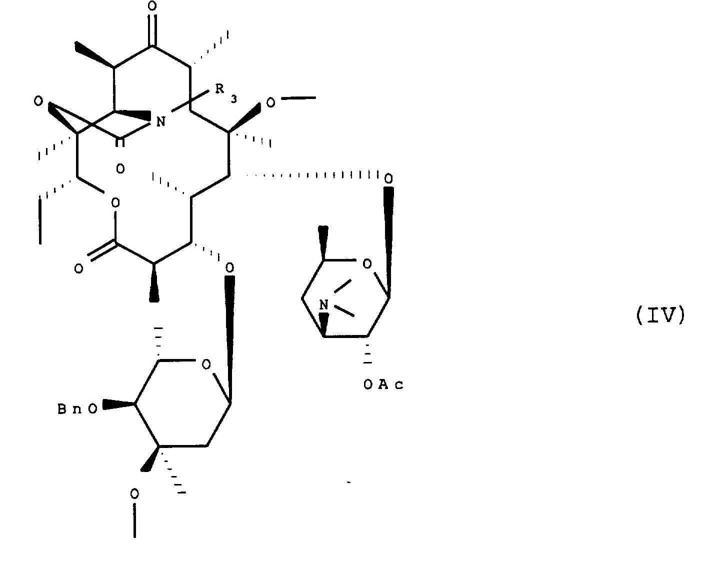 que l'on soumet si désiré à l'action d'un agent de clivage de la fonction ester en 2' pour obtenir le composé de formule (I) dans lequel R₁ est un radical OH, puis soumet si désiré le composé ainsi obtenu à l'action d'un agent de réduction pour effectuer le clivage du groupement benzyloxy carbonyle en 4", l'hydroxyle du groupe OBn et obtenir le produit de formule (I) dans lequel R représente un radical OH puis si désiré soumet le composé de formule (I) à l'action d'un acide pour en former le sel correspondant.

10. A titre de produit chimique nouveau, la 11,12-didéoxy 6-O-méthyl 12,11(oxycarbonyl) (2-hydrazono) érythromycine.

11. A titre de médicaments les composés de formule (I) tels que définis à la revendication 1.

12. A titre de médicaments, le composé de formule (I) tels que définis à la revendication 8.

13. Les compositions pharmaceutiques renfermant comme principe actif au moins un médicament défini à la revendication 11 ou 12.

## Patentansprüche

1. Verbindungen der Formel (I) 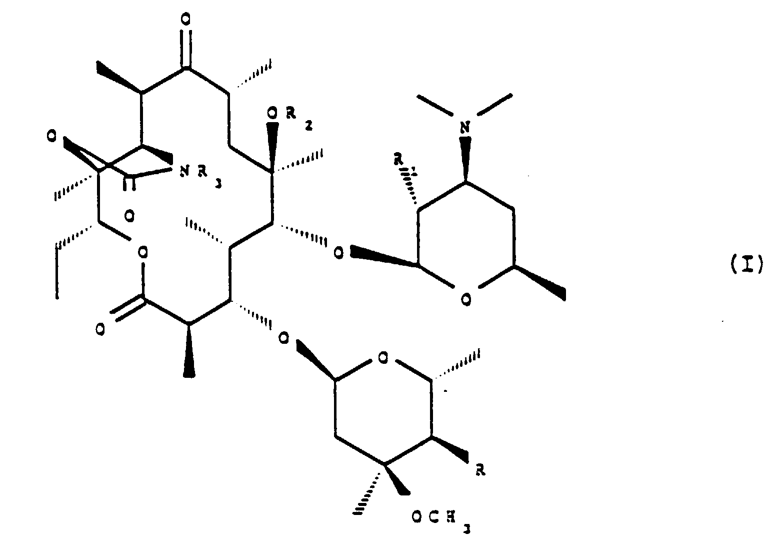 worin
R und R₁ einen Hydroxylrest oder einen O-Acylrest mit 2 bis 20 Kohlenstoffatomen bedeuten,
R₂ für ein Wasserstoffatom oder einen Methylrest steht,
R₃ für einen Rest -N-(CH₂)_{q}R₄ steht, worin q eine ganze Zahl von 0 bis 6 bedeutet, R₄ einen gegebenenfalls substituierten, mono- oder polycyclischen, heterocyclischen Rest darstellt, sowie deren Additionssalze mit Säuren.

2. Verbindungen der Formel (I) gemäß Anspruch 1, worin R und R₁ einen Hydroxylrest bedeuten.

3. Verbindungen der Formel (I) gemäß Anspruch 1 oder 2, worin R₂ einen Methylrest bedeutet.

4. Verbindungen der Formel (I) gemäß Anspruch 3, worin q den Wert 3 besitzt.

5. Verbindungen der Formel (I) gemäß einem der Ansprüche 1 bis 4, worin der heterocyclische Rest R₄ zumindest ein Stickstoffatom umfaßt.

6. Verbindungen der Formel (I) gemäß Anspruch 5, worin der heterocyclische Rest unter den Imidazolyl-, Pyridinyl-, Thiazolyl-, Chinolyl- und Azabenzimidazolyl-Resten, welche gegebenenfalls substituiert sind, ausgewählt sind.

7. Verbindungen der Formel (I) gemäß Anspruch 9, worin R₄ einen 4-Phenyl-1H-imidazolyl- oder 4-Chinolylrest bedeutet.

8. Verbindung der Formel (I) mit der folgenden Bezeichnung:
- 11,12-Didesoxy-6-O-methyl-12,11-(oxycarbonyl-(2-(3-(4-chinolyl)-propyl)-hydrazono))-erythromycin.

9. Verfahren zur Herstellung der Verbindungen der Formel (I) gemäß einem der Ansprüche 1 bis 8, dadurch gekennzeichnet, daß man eine Verbindung der Formel (II) 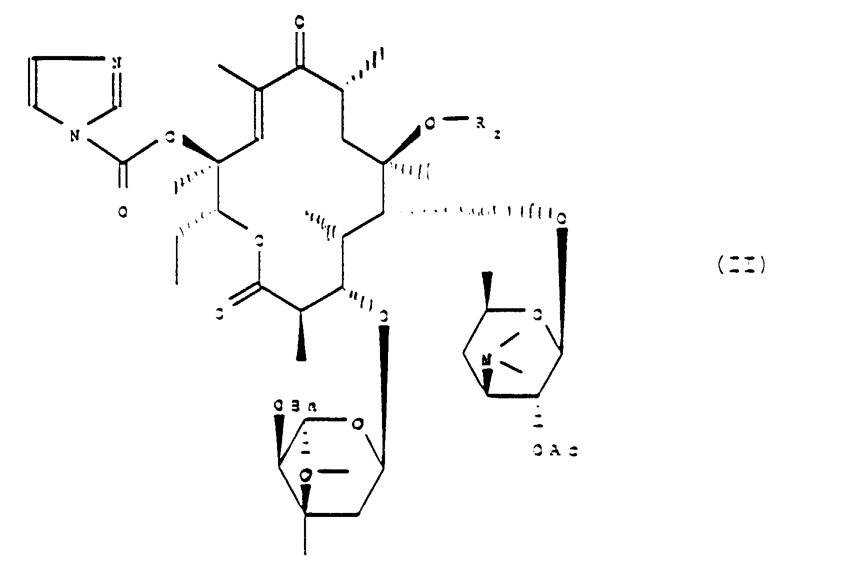 worin R₂ die vorstehende Bedeutung besitzt, Bn einen Benzyloxycarbonylrest bedeutet und Ac für einen Acylrest steht, wie in Anspruch 1 definiert, der Einwirkung einer Verbindung der Formel (III)
R₃NH₂ (III)
worin R₃ die vorstehende Bedeutung besitzt, unterzieht, um zu der Verbindung der Formel (IV) 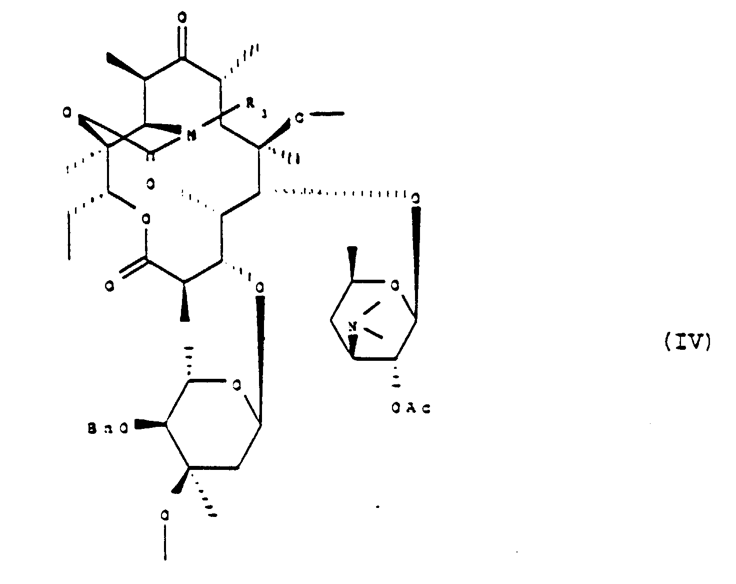 zu gelangen, welche man gewünschtenfalls der Einwirkung eines Mittels zur Abspaltung der Esterfunktion in 2'-Stellung unterzieht, um zu der Verbindung der Formel (I) zu gelangen, worin R₁ ein Rest OH ist, wonach man gewünschtenfalls die so erhaltene Verbindung der Einwirkung eines Reduktionsmittels unterzieht, um die Spaltung der Benzyloxycarbonylgruppe in 4"-Stellung, das Hydroxyl der Gruppe OBn, zu bewirken und das Produkt der Formel (I) zu erhalten, worin R einen Rest OH bedeutet, wonach man gewünschtenfalls die Verbindung der Formel (I) der Einwirkung einer Säure unterzieht, um hieraus das entsprechende Salz zu bilden.

10. Als neues chemisches Produkt das 11,12-Didesoxy-6-O-methyl-12,11-(oxycarbonyl)-(hydrazono)-erythromycin.

11. Als Arzneimittel die Verbindungen der Formel (I) gemäß Anspruch 1.

12. Als Arzneimittel die Verbindung der Formel (I) gemäß Anspruch 8.

13. Pharmazeutische Zusammensetzungen, enthaltend als Wirkstoff zumindest ein Arzneimittel, wie in Anspruch 11 oder 12 definiert.

## Claims

1. The compounds of formula (I): 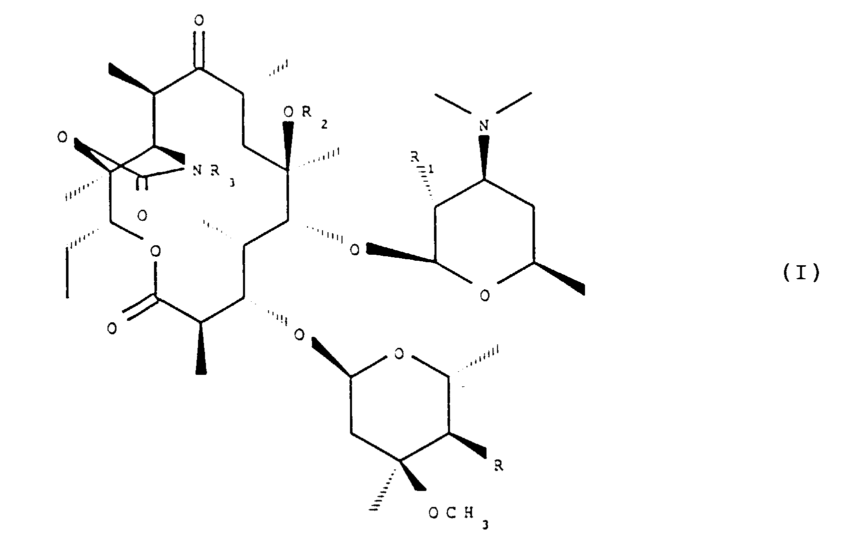 in which R and R₁ represent a hydroxyl or O-acyl radical
containing 2 to 20 carbon atoms,
R₂ is a hydrogen atom or a methyl radical,
R₃ represents an -N-(CH₂)_{q}R₄ radical in which q represents an integer from 0 to 6, R₄ being an optionally substituted mono- or polycyclic heterocyclic radical, as well as their addition salts with acids.

2. The compounds of formula (I) as defined in claim 1, in which R and R₁ represent a hydroxyl radical.

3. The compounds of formula (I) as defined in claim 1 or 2, in which R₂ is a methyl radical.

4. The compounds of formula (I) as defined in claim 3 in which q represents the value 3.

5. The compounds of formula (I) as defined in any one of claims 1 to 4, in which the heterocyclic radical R₄ contains at least one nitrogen atom.

6. The compounds of formula (I) as defined in claim 5, in which the heterocyclic radical is chosen from the group constituted by optionally substituted imidazolyl, pyridinyl, thiazolyl, quinolinyl and azabenzimidazolyl radicals.

7. The compounds of formula (I) as defined in claim 9, in which R₄ represents a 4-phenyl-1H-imidazolyl or 4-quinolinyl radical.

8. The compound of formula (I) the name of which follows:
- 11,12-dideoxy 6-O-methyl 12,11-(oxycarbonyl-(2-(3-(4-quinolinyl) propyl) hydrazono)) erythromycin.

9. Preparation process for the compounds of formula (I) as defined in any one of claims 1 to 8, characterized in that a compound of formula (II): 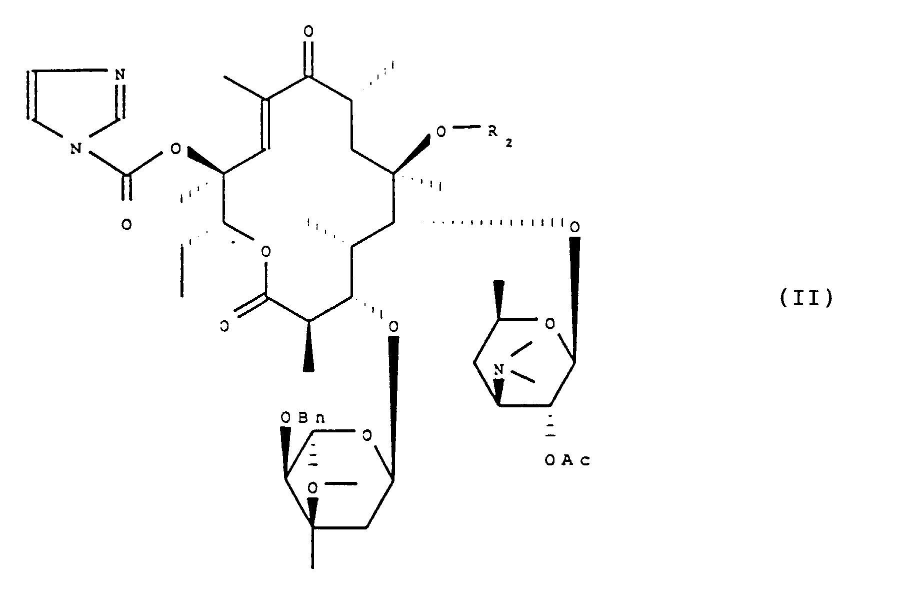 in which R₂ retains its previous meaning, Bn represents a benzyloxycarbonyl radical and Ac an acyl radical, as defined in claim 1 is subjected to the action of a compound of formula (III):
R₃NH₂ (III)
in which R₃ retains its previous meaning in order to obtain the compound of formula (IV): 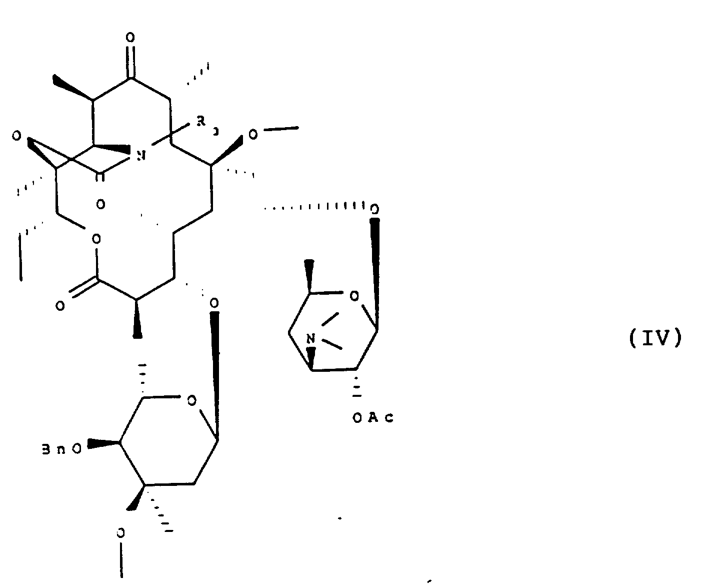 which, if desired, is subjected to the action of a cleavage agent of the ester function in position 2' in order to obtain the compound of formula (I) in which R₁ is an OH radical, then, if desired, the compound thus obtained is subjected to the action of a reducing agent to carry out the cleavage of the benzyloxy carbonyl group in position 4", the hydroxyl of the OBn group and to obtain the product of formula (I) in which R represents an OH radical then, if desired, the compound of formula (I) is subjected to the action of an acid in order to form the corresponding salt.

10. As a new chemical product, 11,12-dideoxy 6-O-methyl 12,11(oxycarbonyl) (2-hydrazono) erythromycin.

11. As medicaments, the compounds of formula (I) as defined in claim 1.

12. As medicaments, the compound of formula (I) as defined in claim 8.

13. The pharmaceutical compositions containing as active principle at least one medicament defined in claim 11 or 12.
